# EUROPÄISCHE PATENTSCHRIFT

(11) **EP 1 651 279 B1**
(45) Veröffentlichungstag und Bekanntmachung des Hinweises auf die Patenterteilung: **09.05.2012**
(21) Anmeldenummer: 04763591.7
(22) Anmeldetag: 29.07.2004
(51) Int. Cl.: A61L 9/012, A61L 9/04, B01D 53/02

(54) **GELARTIGE MASSE IN FLÄCHIGER FORM ZUR BEHANDLUNG VON UMGEBUNGSLUFT**
GEL-LIKE MATERIAL IN A PLANAR FORM FOR THE TREATMENT OF AMBIENT AIR
MASSE GELATINEUSE DE FORME PLATE DESTINEE AU TRAITEMENT DE L'AIR AMBIANT

(30) Priorität: 04.08.2003 DE 10335938
(43) Veröffentlichungstag der Anmeldung: 03.05.2006
(73) Patentinhaber: Guillaume, Bruno, 33400 Talence (FR)
(72) Erfinder: Guillaume, Bruno, 33400 Talence (FR)
(74) Vertreter: Ziegler, Stefan Michael
(86) Internationale Anmeldenummer: PCT/EP2004/008485
(87) Internationale Veröffentlichungsnummer: WO 2005/016392

(56) Entgegenhaltungen:
- EP-A- 0 835 666
- WO-A-98/07454
- FR-A- 2 302 749
- US-A1- 2003 091 529

## Beschreibung

Die Erfindung betrifft eine gelartige Masse zur Behandlung der Umgebungsluft, enthaltend eine Polymermatrix, an die flüchtige aktive Agentien adsorbiert sind, welche freigesetzt werden können und verdunsten und dann mit übelriechenden bzw. schädlichen Substanzen in der Luft reagieren und diese vernichten können.

Zum Entfernen bzw. Reduzieren von übelriechenden Substanzen in der Luft durch Gaswäsche werden in der Praxis verschiedenartige Methoden angewandt. Einige davon nutzen die Fähigkeit von Festsubstanzen mit großer Oberfläche, gasförmige Substanzen zu adsorbieren und damit über einen längeren Zeitraum hinweg zu binden. So werden z.B. mineralische Trägermaterialien, Papiervliese und Aktivkohle als Filtereinsätze zum Entfernen von flüchtigen Verunreinigungen aus Luftund Abluftströmen verwendet.

Ein anderes Prinzip besteht darin, dass man Pulver, saugfähige Papiere, aufnahmefähige Polymere oder hochporöse Tongranulate mit aktiven Agentien, die mit übelriechenden Substanzen reagieren oder diese maskieren können, imprägniert. Sie geben diese aktiven Agentien langsam wieder ab und können dadurch ihre Wirkung entfalten, wenn sie von verunreinigter Luft durch- oder überströmt werden.

In WO-A 01/78794 ist ein wasserfreies Gel zum Beduften oder Desodorieren von Luft beschrieben, welches hergestellt wird durch Vernetzen eines flüssigen, funktionelle Gruppen tragenden Polymeren, vorzugsweise eines maleinisiertes Polybutadiens, mit einem Vernetzungsmittel in Gegenwart eines Duftstoffes oder Desodorants, welches aus dem Gel wieder freigesetzt werden kann.

In US- A 5,569,683 ist ein Gel beschrieben, das eine Polymermatrix aus einem vernetzten Acrylatpolymeren und Duftstoffe enthält, die aus der Matrix wieder freigesetzt werden können.

EP-A-0835666 offenbart eine Vorrichtung zur Freisetzung von Aromen oder desodorierenden Agentien, welche aus einem Gel besteht.

Es hat sich jedoch gezeigt, dass die beschriebenen gelartigen Massen nicht in der Lage sind, die adsorbierten aktiven Agentien über einen längeren Zeitraum hinweg langsam und gleichmäßig wieder freizusetzen.

Der Erfindung lag nun die Aufgabe zugrunde, eine einfach herstellbare, gelartige Masse, die aktive Agentien adsorbiert enthält, bereitzustellen, welche in der Lage ist, diese über einen längeren Zeitraum hinweg langsam und gleichmäßig freizusetzen.

Diese Aufgabe wird durch eine gelartige Masse gemäβ Anspruch 1 gelöst, die eine Matrix aus vernetzten, hydrophile Gruppen tragenden Polymeren enthält, an die 10 bis 90 Gew.%, bezogen auf die Masse, flüchtige aktive Agentien adsorbiert sind, welche mit der Matrix zusammen eine Schwammstruktur bilden, aus der die aktiven Agentien an die Umgebungsluft freigesetzt werden können und verdunsten, wobei die Polymermatrix mindestens zwei verschiedenartigen Polymeren A, B, ... enthält.

Geeignet als vernetzte Polymere sind zwei Polymerklassen, einmal vernetzte (Meth-)Acrylat- Polymere Y und insbesondere vernetzte maleinisierte bzw. epoxidierte Polymere X.

Bevorzugte vernetzte (Meth-)Acrylat- Polymere Y werden hergestellt durch Copolymerisation von monofunktionellen (Meth-)Acrylat- Monomeren mit einem Molekulargewicht von 50 bis 900 und polyfunktionellen (Meth-)Acrylat- Monomeren mit 2 bis 6, vorzugsweise 2 oder 3 polymerisierbaren Doppelbindungen als Vernetzer, wobei das Molverhältnis mono- zu polyfunktionellen Monomeren vorzugsweise 20 : 1 bis 1 : 1, insbesondere 8 :1 bis 1 : 1 beträgt. Es ist wesentlich, dass das vernetzte Polymere hydrophile Gruppen, enthält, beispielsweise HO-, -CH2-CH2-O-, H2N-, -COO- oder CONH- Gruppen. Derartige vernetzte Polymere sind z.B. in der genannten US-A 5,569,683 beschrieben.

Geeignete monofunktionelle Monomere sind z.B. Hydroxyethylacrylat, Acrylamid, Glycerinmonoethylacrylat, Poly(propylenoxid)(ethylenoxid)monomethylacrylat (50 POEP 800 B Blemmer), Polyethylenoxidtetrahydrofuran- monomethacrylat (700 PEP 350 B Blemmer), beide von Nippon Oil and Fats. Geeignete polyfunktionelle Monomere sind Polyethylenglykol- 400- dimethacrylat von Röhm GmbH, Ethylenglykoldimethacrylat, Diethylenglykoldimethacrylat und N,N'-Methylenbisacrylamid von Biorad. Die Herstellung der vernetzten (Meth-) Acrylat- Polymeren erfolgt durch Copolymerisation der Monomeren, wobei die Polymerisation durch Zugabe eines radikalischen Initiators, z.B. Wasserstoffperoxid, und Energiezufuhr, z.B. durch Bestrahlen mit UV- Licht oder durch Erhitzen ausgelöst werden kann.

Die Polymerisation kann in Gegenwart von festen Adsorptionshilfsmitteln, z.B. von Silicagel, Zinkoxid oder Silikaten durchgeführt werden, und insbesondere in Gegenwart von mindestens 0,01 Gew.% Wasser und/oder von anderen quellend wirkenden Flüssigkeiten, wie Alkoholen, Glykolen, Paraffinöl und Polyalkylenoxiden, sowie in Gegenwart der flüssigen aktiven Agentien, wobei eine vorzugsweise offenporige Schwammstruktur entsteht.

Eine andere bevorzugte Polymerklasse X sind vernetzte Umsetzungsprodukte eines hydrophile Gruppen tragenden Polymeren mit einem Vernetzer. Geeignete hydrophile Gruppen tragende Polymere sind maleinisierte oder epoxidierte Polymere, z.B. Umsetzungsprodukte von Polydienen mit einem Molekulargewicht zwischen 400 und 10 000, beispielsweise Polybutadien, Polydecadien und ungesättigte Fettsäuren, wie Soyabohnenöl, mit Maleinsäureanhydrid, ferner Copolymere von Olefinen, wie Ethylen, mit Maleinsäureanhydrid, sowie epoxidiertes Polybutadien und maleinisierter Polymethylvinylether. Auch Kohlehydrate, wie Fructose und Stärke, können als Polymer eingesetzt werden. Besonders bevorzugte Polymere sind Umsetzungsprodukte von flüssigem Polybutadien mit Maleinsäureanhydrid. Bevorzugte Vernetzer sind Polyamine mit 2 oder 3 Aminogruppen, insbesondere Polyoxyalkylenpolyamine, wie Polyoxypropylendiamin und Polyoxypropylentriamin. Neben Polyaminen können auch Harnstoff, Polyethylenimin, Epichlorhydrin, Triethylenglykol und Pyromellithsäuredianhydrid eingesetzt werden.

Auch Zinkoxid, Zinkacetat und Calciumhydroxid können als Vernetzer wirken, wobei letztere wahrscheinlich mit zwei Maleinsäureanhydrid- Gruppen einen stabilen Komplex bilden.
Die Herstellung dieser vernetzten, hydrophile Gruppen tragenden Polymeren erfolgt durch Kondensation des maleinisierten bzw. epoxidierten Polymeren mit dem Polyamin, vorzugsweise in alkoholischer Lösung, insbesondere in Dipropylenglykol, bei erhöhter Temperatur, vorzugsweise zwischen 40° und 60°C. Auch hier ist das bevorzugte Molverhältnis Polymer zu Vernetzer 20 :1 bis 1 :1, insbesondere 8 : 1 bis 1 : 1. Bei einem niedrigen Molverhältnis nahe 1 : 1 erhält man eine starke Vernetzung, was relativ zähe und kompakte gelartige Massen ergibt. Die Kondensation wird in Gegenwart von Zusatzstoffen, wie quellend wirkenden und oberflächenaktiven Flüssigkeiten, vorzugsweise wieder von mindestens 0,01 Gew.% Wasser, und in Gegenwart der aktiven Agentien durchgeführt. Als hydrophile Gruppen wirken insbesondere die von den Polyoxyalkylenpolyaminen stammenden -CRH-O- Gruppen, daneben die Maleinsäureanhydrid- und Carboxyl-Gruppen bzw. die -NR-CO- Gruppen des vernetzten maleinisierten Polymeren, sowie die Epoxidgruppen bzw. -COH-CNR- Gruppen des vernetzten epoxidierten Polymer ren.

In beiden Fällen bilden die vernetzten Polymeren zusammen mit den aktiven Agentien eine vorzugsweise offenzellige Schwammstruktur. Die vernetzten Polymeren weisen ein räumliches Netzwerk mit Poren auf, in dem flüchtige Fremdsubstanzen aufgesaugt und adsorbiert werden können, so dass die Polymeren wie ein Schwamm anquellen. Im angequollenen Zustand besteht das dreidimensionale Netzwerk aus Elementarzellen, die eine Porengröße von 0,1 bis 100 µm, insbesondere von 0,5 bis 50 µm aufweisen.

Erfindungsgemäß enthält die Polymermatrix mindestens zwei verschiedenartige Polymeren A, B, ... .Jedes der Polymeren A, B, ... sollte in der Polymermatrix zu mindestens 10 Gew.%, vorzugsweise von mehr als 20 Gew.%, enthalten sein. Bevorzugt enthält die Polymermischung nur zwei verschiedenartige Polymeren A und B, von denen das eine mehr und das andere weniger wasserverträglich bzw. wasserlöslich ist.

Bei einer bevorzugten Ausführungsform der Erfindung bilden die Polymeren A, B, ... nach der Vernetzung in der Polymermatrix ein interpenetrierendes Netzwerk. Ein solches erhält man, wenn man die Ausgangsmaterialien für die Polymeren A, B, ..., welche der gleichen Polymerklasse X bzw. Y angehören und miteinander verträglich sind, miteinander vermischt und gemeinsam vernetzt, oder wenn man das Ausgangsmaterial für das Polymere B ausreichend lang in Kontakt mit dem bereits vernetzten Polymeren A hält und in dieses eindiffundieren lässt, und erst dann die Vernetzungsreaktion für das Polymere B durchführt.
Ein nur teilweise interpenetrierendes Netzwerk erhält man, wenn man entweder die Ausgangsmaterialien für verschiedenartige Polymerklassen X bzw. Y miteinander vermischt und die Vernetzungsreaktionen für die jeweilige Polymerklasse nacheinander durchführt, oder wenn man bei gleicher Polymerklasse die Ausgangsmaterialien für das Polymer B nur kurz in Kontakt mit dem bereits vernetzten Polymer A bringt, so dass sie nur teilweise eindiffundieren können, und dann vernetzt.
Eine dritte Ausführungsform der Erfindung besteht darin, dass man das Ausgangsmaterial für das Polymer B mit dem bereits vernetzten Polymer A in Kontakt bringt und ohne Wartezeit sofort vernetzt. Man erhält dann eine Polymermatrix, in der die Polymeren A und B in diskreten Schichten nebeneinander vorliegen.

Je nach Anwendungszweck kann die gelartige Masse verschiedenartige aktive Agentien in Mengen von 10 bis 90 Gew.%, vorzugsweise von 20 bis 80 Gew.% enthalten:
- Desodorantien und Duftstoffe, wie Aldehyde, Ketone, Alkohole, Ester, Terpene oder natürliche ölige Essenzen sowie Parfüme;
- Pflanzenschutzmittel, wie Herbizide, Insektizide, Insekten- Repellents, Fungizide, Nematozide oder Akarizide;
- biozid wirkende Substanzen, wie Phenolverbindungen, Halogenverbindungen oder quarternäre Ammoniumverbindungen.

Die aktiven Agentien können entweder dadurch in die gelartige Masse eingebracht werden, dass man sie mit den Ausgangsmaterialien für die Polymeren mischt und in ihrer Gegenwart die Vernetzungsreaktion durchführt, oder indem man sie nachträglich in die Polymermatrix eindiffundieren lässt. Die aktiven Agentien können sowohl hydrophil als auch hydrophob sein.

In einer besonders bevorzugten Ausführungsform enthält die gelartige Masse 0,01 bis 90, vorzugsweise 0,05 bis 60 und insbesondere 0,1 bis 10 Gew.% Wasser. Dieses unterstützt die Bildung der Poren bei der Herstellung der vernetzten Polymeren und sorgt dafür, dass die aktiven Agentien in diese Poren eindringen können, darin festgehalten werden und langsam und gleichmäßig wieder herausdiffundieren. Durch die Menge an Wasser kann die Diffusionsrate kontrolliert werden. Von Bedeutung ist auch, dass die gelartige Masse eine optimale Oberflächenspannung aufweist. Diese liegt bevorzugt zwischen 5 und 80 dynes/cm, insbesondere zwischen 20 und 50 dynes/cm. Die Oberflächenspannung kann mit einem Goniometer zur Bestimmung des Winkels, mit dem ein Tropfen auf der Oberfläche aufliegt, gemessen werden, wie es einem Artikel von Y.C. Ko in Journal of Colloid and Interface Science 1981, 82, Seiten 25- 37, beschrieben ist. Die optimale Oberflächenspannung kann durch die geeignete Kombination von Polymeren, aktiven Agentien und der Menge an zugesetztem Wasser eingestellt werden; sie kann durch einfache Vorversuche ermittelt werden. Je niedriger die Oberflächenspannung ist, desto besser und gleichmäßiger kann das Wasser in der Polymermatrix aufgenommen werden.

Neben den aktiven Agentien und Wasser kann die gelartige Masse noch Flammschutzmittel, wie Bromverbindungen, Zucker oder Azodicarbonamid, ferner Lösungsmittel, wie Alkohole, insbesondere Dipropylenglykol, Mittel zur Verhinderung des Verbackens, wie z.B. Sägemehl, sowie Sublimationshilfsmittel, welche die Freisetzung des aktiven Agens beschleunigen, enthalten.

Eine typische Zusammensetzung der Ausgangsmaterialien bei der Herstellung der bevorzugten gelartigen Masse sieht folgendermaßen aus:

| | |
|---|---|
| Maleinisierte bzw. epoxidierte Polymere: | 10 bis 30 Gew. % |
| Vernetzer: | 0,2 bis 5 Gew. % |
| Wasser: | 0,1 bis 60 Gew.% |
| Flammschutzmittel: | 1 bis 20 Gew.% |
| Aktives Agens: | Rest |

Die gelartige Masse kann als Krümel, Späne, Granulat oder Kugeln zur Anwendung kommen, bevorzugt aber in flächiger Form als Streifen oder Platten mit einer Dicke von 2 bis 50 mm. Diese Streifen oder Platten können auf Netze oder Gitter aufgelegt sein.

Bei einer besonders bevorzugten Ausführungsform ist die gelartige Masse in sehr dünner Schicht auf ein Trägermaterial aufgetragen oder beidseitig von einem Trägermaterial umhüllt, so dass sie mit diesem zusammen ein flächiges Blatt bildet. Das Trägermaterial kann ein Faservlies oder ein Fasergewebe sein, die auch kunststoffbeschichtet sein können. Geeignet sind Vliese und Gewebe auf Basis von Naturfasern, wie Cellulose, Baumwolle oder Leinen, aber auch von synthetischen Fasern, wie Polyamiden oder Polyester. Geeignete Trägermaterialien sind z.B. das Cellulosefaservlies PGI der Fa. Nordlys WDS; das Baumwollgewebe 4110 der FA. Subrenat; ein Faservlies der Fa. Danisco, das mit einem 20 µm dicken Polyoxypropylenfilm und einem 50 µm dicken Polyethylenfilm beschichtet ist; sowie die Textilgewebe K 286, S 434 und S 434 der Fa. Alstrom.
Bei porösen Faservliesen wird die gelartige Masse von dem Vlies aufgenommen, d.h. das Vlies ist mit der gelartigen Masse imprägniert. Bei kunststoffbeschichteten Geweben liegt die gelartige Masse auf dem Trägermaterial auf, wobei die aktiven Agentien durch die Kunststoffbeschichtung hindurch diffundieren können. Durch das Trägermaterial kann die gelartige Masse in sehr dünner Form mechanisch stabilisiert werden, was die Freisetzung der aktiven Agentien begünstigt.
Das Trägermaterial mit der gelartigen Masse liegt dann in Form eines dünnen Blattes, vorzugsweise mit einer Dicke von 0,01 bis 5 mm, insbesondere von 0,1 bis 2 mm vor. Natürlich können mehrere Blätter, z.B. bis zu einer Dicke von 50 mm übereinander gestapelt werden. Die Blätter bzw. Stapel können zusammengerollt werden, wobei die einzelnen Schichten der Rolle zum Beispiel durch flexible Netze oder Gitter voneinander getrennt sind und einen Abstand von 1 bis 100 mm, insbesondere von 2 bis 20 mm voneinander haben. Die Rollen können in rohrförmige Hülsen oder Säulen eingelegt werden, durch die ein Luftstrom parallel zur Längsrichtung der Hülse bzw. Säule entlang der Oberfläche der zusammengerollten Blätter geführt wird, so dass er in möglichst intensiven Kontakt mit der gelartigen Masse treten und die aktiven Agentien freisetzen kann.

Die Hülsen bzw. Säulen können auch an ihrem Umfang eine Vielzahl von Löchern aufweisen, durch die ein Luftstrom senkrecht zur Längsrichtung der Hülse bzw. parallel zur Längsrichtung der Säule geführt werden kann. Derartige Hülsen können quer in den Abluftkanal oder einen Kamin eingesetzt werden. Vorzugsweise senkrecht stehende Säulen können an besonders geruchsbelasteten Stellen von geschlossenen Räumen aufgestellt werden.

Zur Herstellung der dünnen Blätter trägt man auf die Trägerschicht, welche z.B. als Fließband über zwei Rollen geführt wird, erst das Ausgangsmaterial für das Polymere A auf, vernetzt dieses und kalibriert auf die gewünschte Dicke, z.B. mit einem flexiblen Abstreifer. Dann trägt man die Ausgangsmaterialien für das Polymere B auf und vernetzt dieses. Je nach Wartezeit zwischen Auftrag und Vernetzung erhält man ein vollständig interpenetrierendes Netzwerk, ein teilweise interpenetrierendes Netzwerk oder diskrete Schichten der vernetzten Polymeren A und B nebeneinander.
Die aktiven Agentien kann man zusammen mit den Ausgangsmaterialien für die Polymeren zugeben, es ist aber auch möglich, sie in oder auf die Trägermaterialien aufzubringen, von wo sie dann in die sich bildende Polymermatrix eindiffundieren.

Bei einer speziellen Ausführungsform der Erfindung wird die Oberfläche der dünnen Blätter mit ultrafeinen Partikeln einer Halbleiterverbindung belegt, z.B. einem Oxid, Sulfid oder Selenid von Zink oder Cadmium, vorzugsweise von Zinkoxid. Dies kann durch Aufsprühen einer wässrigen Suspension auf die Oberfläche der blattförmigen gelartigen Masse im Anschluss an die Vernetzungsreaktion geschehen, z.B. in Mengen von 0,01 bis 1 g pro cm². Eine Partikelgröße von 1 bis 100 nm, vorzugsweise von 5 bis 60 nm ist bevorzugt. Diese ultrafeinen Halbleiter-Partikel unterstützen die Freisetzung der aktiven Agentien, vor allem aber sind sie in der Lage, mit übelriechenden Substanzen zu reagieren und diese aus der Umgebungsluft zu entfernen.

Aus der erfindungsgemäßen gelartigen Masse werden die aktiven Agentien langsam und gleichmäßig freigesetzt und verdunsten. Es ist von Vorteil, dass die gelartige Masse ihre Wirkung mindestens drei Tage, vorzugsweise mindestens eine Woche und insbesondere einen bis sechs Monate lang beibehält.

Bevorzugt wird die gelartige Masse zum Entfernen bzw. Maskieren von übelriechenden Substanzen aus der Luft eingesetzt, wobei die genannten Desodorantien oder Duftstoffe freigesetzt werden und verdunsten.
Das freigesetzte aktive Agens kann mit den übelriechenden Substanzen in der Gasphase reagieren und dabei die übelriechenden Substanzen aus der Luft entfernen bzw. reduzieren. Neben eigentlichen chemischen Reaktionen, z.B. zwischen Schwefelwasserstoff oder Ammoniak und Aldehyden kann es dabei auch zu Bindungen durch elektrostatische oder van der Waals'sche Kräfte kommen, wodurch die Geruchswahrnehmbarkeit zumindest herabgesetzt wird. Weiterhin können die aktiven Agentien auch die üblen Gerüche maskieren und/oder als spezielle Parfümkomposition den üblen Geruch überdecken und eine Beduftung erzielen.

Folgende praktische Anwendungen sind beispielsweise möglich:

In Tierzuchtanlagen, Ställen, Güllebehältern insbesondere in Schweinemastaniagen, wobei die gelartige Masse in Form von Platten, Streifen oder Blättern auf Gitter oder Netze aufgelegt wird. Diese können direkt an der Decke aufgehängt werden, vorzugsweise werden aber mehrere Gitter übereinander oder nebeneinander auf einem Gestell angeordnet. Man kann auch die oben beschriebenen Rollen aus dünnen Blättern in Hülsen einlegen, durch die die verunreinigte Luft strömen kann. Die Gestelle bzw. Hülsen können in einem Ventilationskasten eingebaut werden, der im Raum oder in Eingangsluftschächten angebracht wird. In letzterem bewirkt die angesaugte Frischluft die Verdunstung der aktiven Agentien.

In Gebäuderäumen, z.B. in Zimmern, Aufenthaltsräumen, Hallen, Küchen und Toiletten von Krankenhäusern, Altersheimen und insbesondere von Hotels und Restaurants. Dabei kann die gelartige Masse wieder in Form von Streifen, Platten oder Blätter auf Gitter oder Netze aufgelegt und in ein Gestell eingebracht oder in Form von Rollen in Hülsen eingelegt werden. Diese können in einen Klimaschacht oder in einen Ventilationskasten eingebracht werden. Ferner kann die gelartige Masse in Säulen eingebracht werden, in denen durch ein Gebläse ein Luftstrom erzeugt wird, welcher die aktiven Agentien freisetzt. Derartige Säulen können an besonders geruchsbelasteten Stellen der Gebäuderäume aufgestellt werden.

In öffentlichen Verkehrsmitteln, wie Eisenbahn-, Straßenbahn-, Untergrundbahnwaggons, Bussen, sowie See- und Luftfahrzeugen. Auch hier kann die gelartige Masse auf Netze oder Gitter aufgelegt und diese übereinander in einem Gestell angeordnet werden oder in Form von Rollen in Hülsen eingelegt werden, die in den Luftschacht des Verkehrsmittels eingebracht werden.

In Industrieanlagen, wobei die gelartige Masse in Abluftkaminen bzw. in Lufteingangsöffnungen oder in die Einblasvorrichtung für Prozessluft angebracht sind. Wo mit starker Verschmutzung zu rechnen ist (z.B. bei Vulkanisationsanlagen) oder wo Abluft mit hoher Temperatur eintritt, welche die Polymermatrix schädigen könnte, kann das aktive Agens in den Raum hinein eingeblasen oder aus einem seitlich angebrachten Einsatz durch Unterdruck eingesaugt werden, wo es sich mit der Abluft vermischen und mit den übelriechenden Substanzen reagieren kann.

In großflächigen Anlagen, z. B. in Klärbecken, Kläranlagen, Kompostieranlagen, Deponien, Müllsortieranlagen, aber auch in ganzen Industriekomplexen, in denen übelriechende feste oder flüssige Stoffe offen gelagert sind. Bevorzugt wird hier die gelartige Masse als.Streifen, Platten oder Stapel von dünnen Blättern in Gitter eingelegt, die zwischen parallele Platten eingebracht werden. Diese können auf Pfosten befestigt werden, die an geeigneten Stellen in der offenen Anlage aufgestellt werden. Der Zwischenraum zwischen den Platten wird von einem Luftstrom durchströmt, der die aktiven Agentien freisetzt, was vorzugsweise durch natürlichen Wind geschieht, der gegebenenfalls durch ein Gebläse verstärkt wird. Eine andere Anwendung der erfindungsgemäßen gelartigen Masse besteht darin, dass sie mit den oben beschriebenen Pflanzenschutzmitteln beladen wird. Sie kann in Form von Krümeln, Spänen oder Granulat direkt auf den Boden oder auf die Pflanzen aufgestreut werden. Platten oder Streifen können in Gitter oder Netze eingelegt und auf Gestelle aufgelegt werden, und dünne Blätter können in Hülsen eingebracht werden.
Die Gestell bzw. Hülsen können im Feld aufgestellt oder im Weinberg an Drähte aufgehängt werden.

Schließlich kann die gelartige Masse auch mit den oben beschriebenen bioziden Agentien beladen werden, die mit schädliche Mikroorganismen in der Luft reagieren und diese vernichten können.

Ein weiterer Gegenstand der Erfindung ist eine gelartige Masse in Form eines extrem dünnen Blattes, enthaltend eine Matrix aus vernetzten, hydrophile Gruppen tragenden Polymeren, an die 10 bis 90 Gew.%, bezogen auf die Masse, flüchtige aktive Agentien adsorbiert sind, die mit der Matrix zusammen eine Schwammstruktur bilden, aus der die aktiven Agentien an die Umgebungsluft freigesetzt werden können und verdunsten. Diese Masse ist dadurch gekennzeichnet, dass sie auf ein Trägermaterial aufgetragen bzw. beidseitig von diesem umhüllt ist und mit dem Trägermaterial zusammen eine Dicke von 0,01 bis 2 mm, vorzugsweise von 0,1 bis 1,8 mm aufweist.

In WO A- 98/07454 ist eine dünne Polymerfolie auf Basis eines Acrylat- Copolymeren beschrieben, die desodorierend wirkt. Das Desodorans ist aber als Comonomer in das Polymere einpolymerisiert und kann daraus nicht freigesetzt werden. Insofern ist der oben beschriebene Gegenstand der Erfindung neu.

Die eben beschriebene gelartige Masse enthält vorzugsweise 0,01 bis 90 Gew.%, insbesondere 0,05 bis 60 Gew.% Wasser. Die dünnen Blätter können wieder in mehreren Lagen übereinander gestapelt werden und zusammengerollt werden.

Derartige extrem dünne Blätter, vor allem wenn die gelartige Masse Wasser enthält, sind auch dann hochwirksam, wenn die Polymermatrix nur ein einziges vernetztes, hydrophile Gruppen tragendes Polymer enthält. Offenbar spielt dabei die relativ große Oberfläche der dünnen Blätter eine Rolle, insbesondere wenn sie mit Abständen zusammengerollt sind.

### Beispiele

### Beispiel 1a

Lösung A: 2 g Acrylamid wurden mit 0,5 g N,N'- Methylen- bis- acrylamid in 7,5 g Wasser, 0,1 ml 30%igem Wasserstoffperoxid und 5 g eines flüssigen Parfüms (Vanillin) vermischt. Die Lösung wurde in einem Ultraschallbad unter schwachem Vakuum entgast und 5 min lang bei 5°C in einer Kolloidmühle mit 300 rpm gerührt. Lösung B: 2 g Acrylamid, 0,5 g Polyethylenglykol-(400)- dimethacrylat in 7,5 g Wasser, 0,1 ml 30%iges Wasserstoffperoxid und 3 g des Parfüms wurden wie oben beschrieben, vermischt, entgast und gerührt.
Stufe 1: Auf ein über zwei Rollen mit einer Geschwindigkeit von 0,1 cm/sec geführtes Fließband aus einem Cellulose- Faservlies (PGI der Fa. Nordlys, WDS) wurde die Lösung A langsam und gleichmäßig aufgetragen, dann wurde mittels einer UV- Lampe (40 mW/cm² bei einem Abstand von 5 cm von dem Trägermaterial) polymerisiert und dann mittels eines flexiblen Abstreifers auf eine Dicke von 1 mm kalibriert.
Stufe 2: Auf das Fließband wurde die Lösung B aufgetragen. Nach einer Wartezeit von 30 min, während der die Lösung B in das bereits gebildete Polymer A eindiffundieren kann, wurde wie oben beschrieben polymerisiert. Insgesamt durchlief das Fließband mit den polymerisierenden Lösungen fünfmal die UV- Lampe. Das gebildete Polymere B war etwas weniger wasserverträglich als das Polymere A; die Polymermatrix enthielt die Polymeren A und B in Form eines interpenetrienden Netzwerkes. Eine Auftrennung der Polymermatrix in zwei Schichten war nicht möglich.
An dem entstandenen, etwas mehr als 1 mm dicken Blatt mit den Abmessungen 22 x 30 cm wurde eine Oberflächenspannung von 45 dynes/cm gemessen. Zwei Lagen des Blattes wurden übereinander gelegt und zu einer vierschichtigen Rolle mit einem Schichtabstand von 5 mm aufgerollt. Die Rolle wurde in einer Feuchtigkeitskammer bei 25 °C und einer Luftfeuchtigkeit von 50 % gelagert. Der Gewichtsverlust durch Freisetzen und Verdunsten des Parfüms und von Wasser wurde gemessen. Die Gewichtsabnahme war gleichmäßig und betrug nach 10 Wochen nur 42 % d.h. dass die Wirksamkeit der gelartigen Masse recht lange anhält.

Zum Vergleich wurden die Lösungen A und B allein polymerisiert und die Gewichts-abnahme gemessen. Der Verlauf der Kurven über die Zeit war ungleichmäßig, nach sechs Wochen betrug der Gewichtsverlust beim Polymer A 23% und beim Polymer B 20%. Danach trat praktisch kein weiterer Gewichtsverlust mehr auf, d.h. die gelartige Masse hatte ihre Wirksamkeit schon nach sechs Wochen verloren. Kurven siehe Abb.

### Beispiel 1 b

Beispiel 1 a wurde wiederholt, die Wartezeit nach Auftragen der Lösung B auf das Polymer A betrug aber nur 10 min. In der entstandenen gelartigen Masse lag die Polymermatrix in Form eines nur teilweise interpenetrierenden Netzwerkes vor. Hier konnten unter hohem Kraftaufwand zwei Schichten mit den Polymeren A bzw. B getrennt werden.

### Beispiel 1 c

Beispiel 1 a wurde wiederholt, wobei aber ohne Wartezeit, unmittelbar im Anschluss an den Auftrag der Lösung B diese vernetzt wurde. In der Polymermatrix lagen die zwei Polymeren A und B als diskrete Schichten nebeneinander vor, die leicht voneinander getrennt werden konnten.

### Beispiel 2

Beispiel 1 wurde wiederholt, wobei aber in Lösung A 4 g Polyethylenoxidmonometh-acrylat (von Nippon Oil and Fat) und 0,5 g Polyethylenglykol-(400)-dimethacrylat eingesetzt wurden, und in Lösung B 5 g Glycerinmonomethacrylat (von Nippon Oil and Fat) und 0,5 g Polyethlenglykol-(400)- dimethacrylat. Das entstandene Polymer A ist weniger wasserverträglich als das Polymer B. Die Oberflächenspannung des Blattes betrug 42 dynes/cm, die Gewichtsabnahme nach 10 Wochen 28 %, die Wirksamkeit ist auch dann noch voll erhalten.

### Beispiel 3

Lösung A: 15 g epoxidiertes Polybutadien (Poly bd von Atofina), 3 g Polyoxypropylen-diamin (Jeffamine D 400 von Huntsman) und 8 g Wasser wurden mit 70 g eines flüssigen Parfüms vermischt, wie in Beispiel 1 entgast und gerührt.
Lösung B: 20 g Fructose (Arabino-2-hexulose von Cerestar) und 1 g Pyromellithsäure-dianhydrid (ALLCO PDMA von Allco) wurden mit 10 g eines flüssigen Insektizids (Azadirachtin A von Andermatt) und 0,3 g Wasser vermischt, entgast und gerührt.
Die Stufen 1 und 2 der Vernetzungsreaktion wurden wie in Beispiel 1 durchgeführt, jedoch mit einem 80°C heißen Luftstrom statt der UV- Lampe. Das entstandene Polymer A ist nicht wasserlöslich, wohl aber das Polymer B. Die Oberflächenspannung betrug 35 dynes/cm, die Gewichtsabnahme nach 10 Wochen 25 %.
Anwendung: Zehn Lagen des erhaltenen, etwas mehr als 1 mm dicken Blattes wurden übereinandergelegt und in ein Netz eingebracht. Mehrere solcher Netze wurden an die Drahtkonstruktion eines Weinbergs angehängt, wo sie über drei Monate hinweg ihre insektizide Wirkung entfalteten.

### Beispiel 4

Lösung A: 16 g maleinisierter Polyvinylmethylether (Gantrez SP 215 der Fa. ISP), 0,5 g Polyoxypropylendiamin, 0,5 g Triethylenglykoldiamin 70 g eines flüssigen Parfüms, 1 g maleinisiertes Soyabohnenöl und 0,3 g Wasser wurden gemischt. Die Mischung wurde entgast und gerührt.
Lösung B: 0,5 g Polyoxypropylendiamin, 0,5 g Triethylenglykoldiamin, 30 g Parfüm, 40 g maleinisiertes Soyabohnenöl und 1 g Pyromellithsäuredianhydrid wurden vermischt, entgast und gerührt. Die Lösungen wurden wie in Beispiel 3 vernetzt.
Polymer A ist wasserlöslich, Polymer B wasserunlöslich. Die Gewichtsabnahme nach 10 Wochen betrug 20 %. Dieser niedrige Wert ist vermutlich auf den geringen Gehalt der gelförmigen Masse an Wasser zurückzuführen.

### Beispiel 5

Beispiel 1 wurde wiederholt, wobei aber das flüssige Parfüm nicht den Ausgangsmaterialien für die Polymeren A und B zugesetzt wurde, sondern auf das Faservlies aufgetragen und von diesem aufgesaugt wurde. Während des Auftrags der Ausgangsmaterialien für die Polymeren und im Verlauf der Vernetzungsreaktion diffundierte das Parfüm in die gelartige Masse ein.

### Beispiel 6

Beispiel 1 wurde wiederholt, wobei aber der Lösung A 0,05 Gew.% Zinkoxid zugesetzt wurde. Dieses wirkt als zusätzliches Vernetzungsmittel, außerdem sind Zinkund Calciumverbindungen in der Lage, störende Geruchsstoffe aus der Luft, z.B. Schwefelverbindungen, zu binden.

### Beispiel 7

Lösung A: 10 g maleinisiertes Polybutadien (Umsetzungsprodukt aus flüssigem Polybutadien mit Maleinsäureanhydrid - LITHENE der Fa. Revertex) wurden bei 50°C mit 40 g Dipropylenglykol vermischt; und 3 g Polyoxypropylendiamin (MG 400) wurden bei Raumtemperatur mit 45 g Dipropylenglykol vermischt. Die beiden Mischungen wurden in einem flachen Gefäß bei 20°C zusammengerührt. Lösung B: 16 g des Ethylhalbesters der Polycarbonsäure aus maleinisiertem Vinylmethylether (Gantrez SP 215 von ISP), 0,5 g Polyoxypropylendiamin, 0,5 g Polyoxypropylentriamin (Molverhältnis 1 : 1) und 3 g Wasser wurden mit 70 g einer 50%igen Lösung eines aktiven Agens (Vanillin) in Diethylenglykol bei 20 °C vermischt.
Vernetzung: Die Lösungen A und B wurden miteinander vermischt und 40 min lang bei 80 °C gerührt. Die entstandene gelartige Masse enthält als Polymermatrix ein teilweise interpenetrierendes Netzwerk aus den Polymeren A und B.

Anwendung: Die gelartige Masse wurde in Platten von 2 cm Dicke, 20 cm Breite und 20 cm Länge geschnitten und auf ein Metallnetz aufgelegt. Dieses Netz wurde zwischen zwei parallele, 50 x 50 cm große Kunststoffplatten mit einem Abstand von 10 cm eingebracht. 16 derartiger Platten wurden an der schrägen Einfassung eines 100 m² großen Klärbeckens aufgelegt, wo sie über mehrere Wochen hinweg ihre desodorierende Wirkung entfalteten.

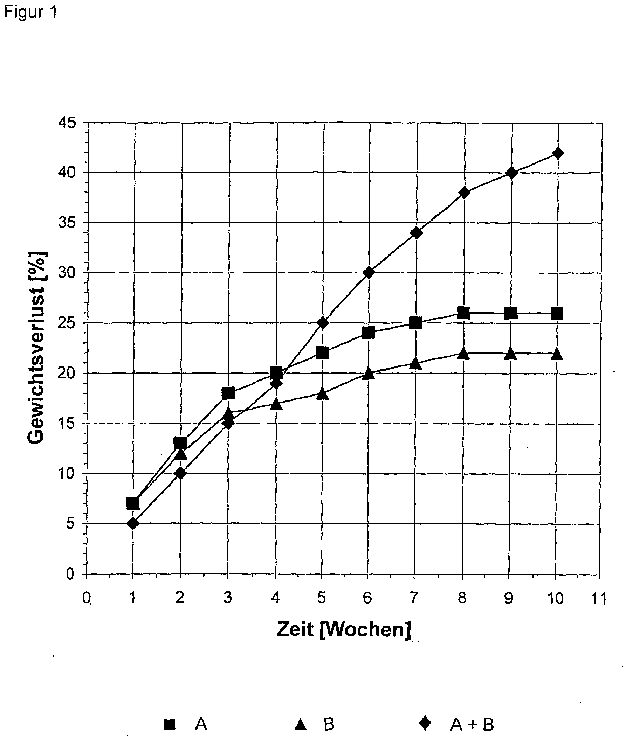

## Patentansprüche

1. Gelartige Masse zur Behandlung der Umgebungsluft, enthaltend eine Matrix aus vernetzten, hydrophile Gruppen tragenden Polymeren, nämlich
X) Umsetzungsprodukten eines hydrophile Gruppen tragenden Polymeren mit einem Molekulargewicht von mehr als 400, ausgewählt aus maleinisierten oder epoxidierten Polymeren oder Kohlehydraten, mit einem Vernetzer, ausgewählt aus Polyaminen mit 2 oder 3 Aminogruppen, Harnstoff, Polyethylenimin, Epichlorhydrin, Triethylenglykol, Pyromellithsäuredianhydrid, Zinkoxid, Zinkacetat oder Calciumhydroxid,
oder
Y) Copolymerisaten aus einem monofunktionellen (Meth-) AcrylatMonomeren mit einem Molekulargewicht von 50 bis 1000 und einem polyfunktionellen (Meth-) Acrylat- Monomeren als Vernetzer,
an die 10 bis 90 Gew.%, bezogen auf die Masse, flüchtige aktive Agentien adsorbiert sind, die mit der Matrix zusammen eine Schwammstruktur bilden, aus der die aktiven Agentien an die Umgebungsluft freigesetzt werden können und verdunsten,
**dadurch gekennzeichnet, dass** die Polymermatrix mindestens zwei verschiedenartige Polymere A, B, ... enthält.

2. Gelartige Masse nach Anspruch 1, **dadurch gekennzeichnet, dass** die Polymeren A, B, ... nach der Vernetzung in der Polymermatrix ein interpenetrierendes Netzwerk bilden.

3. Gelartige Masse nach Anspruch 1, **dadurch gekennzeichnet, dass** die Polymeren A, B, ... nach der Vernetzung in der Polymermatrix ein teilweise interpenetrierendes Netzwerk bilden.

4. Gelartige Masse nach Anspruch 1, **dadurch gekennzeichnet, dass** die Polymeren A, B, ... nach der Vernetzung in der Polymermatrix in diskreten Schichten nebeneinander vorliegen.

5. Gelartige Masse nach Anspruch 1, **dadurch gekennzeichnet, dass** die Polymermatrix aus zwei verschiedenartigen Polymeren A und B besteht, von denen das eine mehr und das andere weniger wasserverträglich ist.

6. Gelartige Masse nach Anspruch 1, **dadurch gekennzeichnet, dass** jedes Polymere A, B ... in der Polymermatrix zu mindestens 10 Gew.% enthalten ist.

7. Gelartige Masse nach Anspruch 1, **dadurch gekennzeichnet, dass** der Vernetzer der Polymerklasse X ein Polyamin, vorzugsweise Polyoxypropylendiamin oder Polyoxypropylentriamin, ist und das hydrophile Gruppen tragende Polymer ein maleinisiertes oder epoxidiertes Polydiolefin, vorzugsweise Polybutadien oder Polydecadien, ist.

8. Gelartige Masse nach Anspruch 1, **dadurch gekennzeichnet, dass** sie auf einem Trägermaterial aufgetragen oder beidseitig von einem solchen umhüllt ist, so dass sie mit dem Trägermaterial zusammen in Form eines flächigen Blattes vorliegt.

9. Gelartige Masse nach Anspruch 1, **dadurch gekennzeichnet, dass** sie in Form von Streifen oder Platten mit einer Dicke von 2 bis 50 mm zur Anwendung kommt, die auf Gitter oder Netze aufgelegt sind.

10. Verwendung der gelartigen Masse nach Anspruch 1 zum Entfernen bzw.
Maskieren von übelriechenden Substanzen aus der Luft, **dadurch gekennzeichnet, dass** die adsorbierten aktiven Agentien langsam und gleichmäßig freigesetzt werden und verdunsten und dann mit den übelriechenden Substanzen reagieren oder diese maskieren.

11. Verwendung der gelartigen Masse nach Anspruch 1 zum Pflanzenschutz, **dadurch gekennzeichnet, dass** die adsorbierten aktiven Agentien langsam und gleichmäßig freigesetzt werden und ihre Wirksamkeit gegen die Schädlinge bzw. Schadstoffe entfalten.

12. Verwendung der gelartigen Masse nach Anspruch 1 zum Vernichten von schädlichen Mikroorganismen, **dadurch gekennzeichnet, dass** die adsorbierten aktiven Agentien langsam und gleichmäßig freigesetzt werden und mit den Mikroorganismen reagieren und diese vernichten.

## Claims

1. Gel-like material for the treatment of ambient air, comprising a matrix of crosslinked polymers carrying hydrophilic groups, namely
X) reaction products of a polymer carrying hydrophilic groups and having a molecular weight of more than 400, selected from maleized or epoxidized polymers or carbohydrates, with a crosslinker selected from polyamines having 2 or 3 amino groups, urea, polyethyleneimine, epichlorohydrin, triethylene glycol, pyromellitic dianhydride, zinc oxide, zinc acetate or calcium hydroxide,
or
Y) copolymers of a monofunctional (meth)acrylate monomer having a molecular weight of 50 to 1000 and a polyfunctional (meth)acrylate monomer as crosslinker,
to which 10 to 90% by weight, based on the material, of volatile active agents are adsorbed which, together with the matrix, form a sponge structure from which the active agents can be released into the ambient air and vaporize, **characterized in that** the polymer matrix comprises at least two different polymers A, B, ....

2. Gel-like material according to Claim 1, **characterized in that** the polymers A, B, ... form an interpenetrating network following crosslinking in the polymer matrix.

3. Gel-like material according to Claim 1, **characterized in that** the polymers A, B, ... form a partially interpenetrating network following crosslinking in the polymer matrix.

4. Gel-like material according to Claim 1, **characterized in that** the polymers A, B, ... are present in discrete layers alongside one another following crosslinking in the polymer matrix.

5. Gel-like material according to Claim 1, **characterized in that** the polymer matrix consists of two different polymers A and B, of which one is more water-compatible and the other is less water-compatible.

6. Gel-like material according to Claim 1, **characterized in that** each polymer A, B ... is present in the polymer matrix to at least 10 by weight.

7. Gel-like material according to Claim 1, **characterized in that** the crosslinker of the polymer class X is a polyamine, preferably polyoxypropylenediamine or polyoxypropylenetriamine, and the polymer carrying hydrophilic groups is a maleized or epoxidized polydiolefin, preferably polybutadiene or polydecadiene.

8. Gel-like material according to Claim 1, **characterized in that** it is applied to a support material or is surrounded on both sides by such a material such that it is present, together with the support material, in the form of a flat sheet.

9. Gel-like material according to Claim 1, **characterized in that** it is used in the form of strips or sheets with a thickness of 2 to 50 mm which are placed onto grids or meshes.

10. Use of the gel-like material according to Claim 1 for removing and/or masking foul-smelling substances from the air, **characterized in that** the adsorbed active agents are released slowly and uniformly and vaporize and then react with the foul-smelling substances or mask them.

11. Use of the gel-like material according to Claim 1 for crop protection, **characterized in that** the adsorbed active agents are released slowly and uniformly and develop their effectiveness against the pests and/or harmful substances.

12. Use of the gel-like material according to Claim 1 for destroying harmful microorganisms, **characterized in that** the adsorbed active agents are released slowly and uniformly and react with the microorganisms and destroy them.

## Revendications

1. Masse de type gel destinée au traitement de l'air ambiant, contenant une matrice de polymères réticulés portant des groupes hydrophiles, à savoir
X) des produits de la réaction d'un polymère portant des groupes hydrophiles et présentant une masse moléculaire supérieure à 400, sélectionné parmi des polymères ou des hydrates de carbone maléinisés ou époxydés, avec un agent de réticulation sélectionné parmi des polyamines comportant 2 ou 3 groupes amino, une urée, une polyéthylèneimine, une épichlorhydrine, du triéthylèneglycol, un dianhydride d'acide pyromellitique, de l'oxyde de zinc, de l'acétate de zinc ou de l'hydroxyde de calcium, ou
Y) des copolymérisats d'un monomère monofonctionnel d'acrylate ou de méthacrylate présentant une masse moléculaire de 50 à 1000 et d'un monomère polyfonctionnel d'acrylate ou de méthacrylate en tant qu'agent de réticulation,
au niveau de laquelle sont adsorbés 10 à 90 % en poids, sur la base de la masse, d'agents actifs volatils, qui forment avec la matrice une structure spongieuse à partir de laquelle les agents actifs peuvent être libérés dans l'air ambiant et s'évaporer,
**caractérisée en ce que** la matrice polymère contient au moins deux polymères différents A, B, ....

2. Masse de type gel selon la revendication 1, **caractérisée en ce que** les polymères A, B, ... suite à la réticulation forment dans la matrice polymère un réseau interpénétrant.

3. Masse de type gel selon la revendication 1, **caractérisée en ce que** les polymères A, B, ... suite à la réticulation forment dans la matrice polymère un réseau partiellement interpénétrant.

4. Masse de type gel selon la revendication 1, **caractérisée en ce que** les polymères A, B, ... suite à la réticulation se présentent dans la matrice polymère en couches discrètes les unes à côté des autres.

5. Masse de type gel selon la revendication 1 , **caractérisée en ce que** la matrice polymère se compose de deux polymères A et B différents, parmi lesquels un est plus compatible avec l'eau et l'autre moins compatible avec l'eau.

6. Masse de type gel selon la revendication 1, **caractérisée en ce que** chaque polymère A, B, ... est contenu dans la matrice polymère à raison d'au moins 10 % en poids.

7. Masse de type gel selon la revendication 1, **caractérisée en ce que** l'agent de réticulation de la classe de polymères X est une polyamine, de préférence une polyoxypropylènediamine ou une polyoxypropylènetriamine, et **en ce que** le polymère portant des groupes hydrophiles est une polydioléfine maléinisée ou époxydée, de préférence le polybutadiène ou le polydécadiène.

8. Masse de type gel selon la revendication 1, **caractérisée en ce qu'**elle est appliquée sur un matériau de support ou enveloppée par celui-ci des deux côtés de façon telle qu'elle se présente avec le matériau de support sous la forme d'une plaque plate.

9. Masse de type gel selon la revendication 1, **caractérisée en ce qu'**elle est utilisée sous la forme de bandes ou de plaques, d'une épaisseur de 2 à 50 mm, qui sont appliquées sur des grilles ou treillis.

10. Utilisation de la masse de type gel selon la revendication 1 pour éliminer ou masquer des substances malodorantes de l'air, **caractérisée en ce que** les agents actifs adsorbés sont libérés et s'évaporent lentement et régulièrement puis réagissent ensuite avec les substances malodorantes ou masquent celles-ci.

11. Utilisation de la masse de type gel selon la revendication 1 pour la protection des plantes, **caractérisée en ce que** les agents actifs adsorbés sont libérés lentement et régulièrement puis déploient leur efficacité contre des nuisibles ou des substances nocives.

12. Utilisation de la masse de type gel selon la revendication 1 pour détruire des microorganismes nuisibles, **caractérisée en ce que** les agents actifs adsorbés sont libérés lentement et régulièrement et réagissent avec les microorganismes et les détruisent.
